# EUROPÄISCHE PATENTANMELDUNG

(11) **EP 2 719 765 A1**
(43) Veröffentlichungstag der Anmeldung: **16.04.2014**
(21) Anmeldenummer: 12007064.4
(22) Anmeldetag: 11.10.2012
(51) Int. Cl.: C12N 15/88

(54) **Ex vivo-Verfahren zur Einbringung von Nukleinsäuren in Zellen**

(71) Anmelder: Secutech International Pte. Ltd., Singapore 069443 (SG)
(72) Erfinder: Kosak, Hans, Dr., 53115 Bonn (DE); Weichert, Marcus, 13593 Berlin (DE)
(74) Vertreter: Graf von Stosch, Andreas

(57) **Zusammenfassung**

Gegenstand der vorliegenden Anmeldung sind ex vivo (Transfektions-)Verfahren zur Einbringung von Nukleinsäuren in Zellen und/oder Zellgewebe. Dafür wird eine Zusammensetzung, die mindestens ein organisches Lösungsmittel umfasst, in dem Komplexe aus mindestens einer Nukleinsäure und mindestens einem organischen Kation gelöst vorliegen mit den Zellen und/oder Zellgeweben in Kontakt gebracht. Die Nukleinsäuren sind in der Regel derart komplexiert, dass die negativen Ladungen der Nukleinsäuren durch positive Ladungen der organischen Kationen im Wesentlichen vollständig kompensiert sind.

## Beschreibung

Gegenstand der vorliegenden Anmeldung sind ex vivo (Transfektions-)Verfahren zur Einbringung von Nukleinsäuren in Zellen und/oder Zellgewebe. Dafür wird eine Zusammensetzung, die mindestens ein organisches Lösungsmittel umfasst, in dem Komplexe aus mindestens einer Nukleinsäure und mindestens einem organischen Kation gelöst vorliegen mit den Zellen und/oder Zellgeweben in Kontakt gebracht. Die Nukleinsäuren sind in der Regel derart komplexiert, dass die negativen Ladungen der Nukleinsäuren durch positive Ladungen der organischen Kationen im Wesentlichen vollständig kompensiert sind.

### Stand der Technik

Die Einbringung von Nukleinsäuren in Zellen ist in der Molekularbiologie und Medizin von großem Interesse. Durch das Einbringen von Nukleinsäure wie z.B. siRNAs lassen sich praktisch alle Zellfunktionen gezielt manipulieren und studieren. Aktuell am meisten verbreitet sind Transfektionsverfahren auf der Basis von Liposomen. Dabei werden zumeist kationische Verbindungen in eine wässrige Flüssigkeit eingeführt, in der die Verbindungen nicht frei löslich sind und Liposomen mit einer positiven Außenlandung bilden. Zu dieser Suspension werden Nukleinsäuren gegeben, die aufgrund ihrer negativen Ladung ungeordnet an die Oberfläche der Liposomen binden. Das Verhältnis zwischen den positiven Ladungen der Liposomen und den negativen Ladungen der Nukleinsäuren ist dabei so gewählt, das ein Überschuss an positiven Ladungen bestehen bleibt. Die entstandene Suspension wird nun mit den Zellen z.B. Zellen in einem Zellkulturmedium gegeben. Die Liposomen binden aufgrund der Bereiche mit positiven Ladungsüberschuss an die negativ geladene Membran der Zellen und werden mit der an ihnen haftenden Nukleinsäure von den Zellen aufgenommen.

Die gängigen Liposomenverfahren haben einige Nachteile. Werden zum Beispiel die Nukleinsäuren in wässriger Lösung mit den Liposomen in Kontakt gebracht, so muss ein erheblicher Aufwand zum Schutz der Nukleinsäuren vor dem Abbau durch Nukleasen betrieben werden. Insbesondere beim Arbeiten mit RNA, z.B. siRNA, ist der Aufwand besonders groß, da ubiquitär vorkommende RNAsen nur sehr schwer inaktiviert werden können. Ferner muss der Kontakt der Nukleinsäuren mit dem Liposomen unmittelbar vor der Verwendung, d.h. der Zugabe zu den Zellen, hergestellt werden. Das hat den Nachteil, dass keine länger stabilen Formulierungen der Nukleinsäuren möglich sind. Weiterhin sind bei diesem Verfahren eine Vielzahl von Pipettierschritten notwendig, die aufwendig und eine Quelle möglicher Fehler sind. Auch die Zellmedien, mit denen die Liposomen gemischt werden, müssen häufig frei von Proteinen und Antibiotika sein, da sich sonst leicht inhibierenden Aggregate von Proteinen oder Antibiotika mit den Liposomen bilden könnten. Schließlich führt der Überschuss an positiven Ladungen der Liposomen zu einer Destabilisierung der Zellmembran, so dass die Liposomen oft cytotoxisch wirken.

Es besteht daher weiterhin ein Bedarf an neuen Verfahren zur Einbringung von Nukleinsäuren in Zellen, die vorzugsweise die oben genannten Nachteile ganz oder zumindest teilweise überwinden.

### Zusammenfassung der Erfindung

Die vorliegende Erfindung betrifft ein ex vivo Verfahren zur Einbringung von Nukleinsäuren in Zellen und/oder Zellgewebe, wobei das Verfahren folgende Schritte umfasst:
a) Bereitstellen einer Zusammensetzung, die mindestens ein organisches Lösungsmittel umfasst, in dem Komplexe aus mindestens einer Nukleinsäure und mindestens einem organischen Kation gelöst vorliegen, und
b) In-Kontakt-Bringen der Zusammensetzung nach a) ex vivo mit Zellen und/oder Zellgewebe.

Die Verwendung der in einem organischen Lösungsmittel gelösten komplexierten Nukleinsäuren im Vergleich beispielsweise zu Liposomen kann die Transfektionszusammensetzung Tage oder sogar Wochen vor der eigentlichen Transfektion vorformuliert werden. Zudem sind die Nukleinsäuren in dieser Formulierung weitgehend gegenüber nukleolytischem Abbau geschützt. Dies ist insbesondere von Bedeutung, wenn RNA transfiziert werden soll. Die Formulierung kann überdies bei Raumtemperatur oder gekühlt über Tage und Wochen gelagert werden. Das erfinderische Verfahren ermöglicht auch eine Einbringung von Nukleinsäuren in Gegenwart von Medien, die z.B. Proteine oder Antibiotika enthalten. Da ferner bei der Herstellung der Nukleinsäurekomplexe ein etwaiger Überschuss an organischen Kationen abgetrennt werden kann, fällt auch die diesbezügliche cytotoxische Wirkung, wie sie bei Liposomen beobachtet wird, geringer aus.

### Detaillierte Beschreibung der Erfindung

In den erfindungsgemäßen Verfahren werden Zusammensetzungen benutzt, die mindestens ein organisches Lösungsmittel umfassen. Das organische Lösungsmittel enthält darin gelöst eine oder mehrere mit einem oder mehreren organischen Kationen komplexierte, Nukleinsäuren. Die Nukleinsäuren sind in der Regel derart komplexiert, dass die negativen Ladungen der Nukleinsäuren durch positive Ladungen der organischen Kationen im Wesentlichen vollständig kompensiert sind. Im Wesentlichen vollständig kompensiert bedeutet hier insbesondere, dass das Verhältnis der Anzahl an positiven Ladungen der Kationen zu negativen Ladungen der Nukleinsäure vorzugsweise größer oder gleich 0,5 ist, vorzugsweise größer oder gleich 0,6 ist, vorzugsweise größer oder gleich 0,7 ist, vorzugsweise größer oder gleich 0,8 ist, vorzugsweise größer oder gleich 0,9 ist, vorzugsweise größer oder gleich 0,91 ist, vorzugsweise größer oder gleich 0,92 ist, vorzugsweise größer oder gleich 0,93 ist, vorzugsweise größer oder gleich 0,94 ist, vorzugsweise größer oder gleich 0,95 ist, vorzugsweise größer oder gleich 0,96 ist, vorzugsweise größer oder gleich 0,97 ist, vorzugsweise größer oder gleich 0,98 ist, vorzugsweise größer oder gleich 0,985 ist, vorzugsweise größer oder gleich 0,99 ist, vorzugsweise größer oder gleich 0,995 ist, oder sogar gleich 1 ist. Das Verhältnis der Anzahl an positiven Ladungen der Kationen zu negativen Ladungen der Nukleinsäure kann beispielsweise im Bereich 0,5 - 2 liegen, z.B. im Bereich 0,6 - 1,8 liegen, im Bereich 0,7 - 1,3, im Bereich 0,8 - 1,2 liegen, im Bereich 0,9 - 1,1 liegen, im Bereich 0,91 - 1,09 liegen, im Bereich 0,92 - 1,08 liegen, im Bereich 0,93 - 1,07 liegen , im Bereich 0,94 - 1,06 liegen, im Bereich 0,95 - 1,05 liegen, im Bereich 0,96 - 1,04 liegen, im Bereich 0,96 - 1,04 liegen, im Bereich 0,97 - 1,03 liegen, im Bereich 0,98 - 1,02 liegen, im Bereich 0,99 - 1,01 liegen, im Bereich 0,995 - 1,005 liegen, oder sogar gleich 1 sein. Letzterer Wert wird insbesondere dann erzielt, wenn bei der Herstellung (siehe später) Kationen bei der Fällung der Nukleinsäure im Überschuss vorliegen und das Präzipitat aus organischen Kation und Nukleinsäure mit Wasser gewaschen wird (siehe später). Die Zusammensetzung nach a) wird dann im Wesentlichen keine freien, nicht komplexierten organischen Kationen umfassen.

Es wurde festgestellt, dass es durch diese Form der Komplexierung möglich ist, Nukleinsäuren, die ansonsten in organischen Lösungsmitteln nicht löslich sind, in hohen Mengen in solche Lösungsmittel einzubringen. Nukleinsäuren, wie z.B. DNA oder RNA, sind unter physiologischen Bedingungen anionische Polymere, die über eine hohe Anzahl von negativ geladenen Phosphat-Gruppen verfügen. Aufgrund der Ladung der Polymere sind diese in wässriger Lösung gut löslich, während die Löslichkeit in organischen Lösungsmitteln äußerst gering ist. Es konnte gezeigt werden, dass sich die in organischen Lösungsmitteln gelösten, komplexierten Nukleinsäuren in wirksamer Weise in Zellen und/oder Gewebe z.B. eines Säugetiers ex vivo einbringen lassen.

"Mindestens ein organisches Kation" bedeutet, dass mindestens ein Typ an organischem Kation vorliegt (z.B. Cetyltrimethylammoniumionen, Dimethyldioctadecylammoniumionen, oder Benzalkoniumionen). Es können aber auch Kationengemische von zwei oder mehr, drei oder mehr, vier oder mehr, fünf oder mehr, etc. unterschiedlichen organischen Kationen vorliegen (z.B. Cetyltrimethylammoniumionen neben Dimethyldioctadecylammoniumionen). Üblicherweise ist das mindestens eine organische Kation einfach positiv geladen.

"Mindestens ein organisches Lösungsmittel" bedeutet, dass mindestens ein organisches Lösungsmittel vorliegt. Es können aber auch Lösungsmittelgemische von zwei oder mehr, drei oder mehr, vier oder mehr, fünf oder mehr, etc. unterschiedlichen organischen Lösungsmitteln vorliegen. Die Nukleinsäuren sind dann entsprechend in diesen Lösungsmittelgemischen gelöst. Der Begriff soll auch nicht ausschließen, dass ein Gemisch (mindestens) eines organischen Lösungsmittels mit einer wässrigen Flüssigkeit vorliegt. Organische Flüssigkeiten wie Alkohole, in denen die hier beschriebenen Komplexe löslich sind, lassen sich mit Wasser beliebig mischen und bilden einen homogene Phase. Daher können die Komplexe über die Lösung in derartigen organischen Lösungsmitteln auch in wässrige Medien eingebracht werden. Die Nukleinsäuren sind dann entsprechend in diesem Lösungsmittelgemisch aus (mindestens) einem organischen Lösungsmittel und Wasser gelöst. Mit anderen Worten: erfindungsgemäß liegen die Komplexe dann in der Zusammensetzung in einer homogenen Phase gelöst vor. Diese homogene Phase wiederum umfasst mindestens ein organisches Lösungsmittel.

"Mindestens eine Nukleinsäure" bedeutet, dass mindestens eine Nukleinsäure vorliegt. Es können aber auch Nukleinsäuregemische von zwei oder mehr, drei oder mehr, vier oder mehr, fünf oder mehr, etc. unterschiedlichen Nukleinsäuren vorliegen. Bei derartigen Nukleinsäuregemischen kann die Art der Nukleinsäure unterschiedlich sein, z.B. eine Mischung von DNA und RNA, es kann aber auch lediglich ein Unterschied in der Sequenz vorliegen.

Die mindestens eine Nukleinsäure löst sich in den organischen Lösungsmitteln aufgrund der Ladungskompensation in den gebildeten Komplexen. Die Lösung der Nukleinsäure benötigt daher nicht die Bildung von Micellen, Liposomen oder ähnlichen nanopartikulären Strukturen. Üblicherweise liegen daher Komplexe aus mindestens einer Nukleinsäure und mindestens einem organischen Kation gelöst vor, wobei die Komplexe sich nicht im Inneren einer Micelle, eines Liposoms oder ähnlicher nanopartikulärer Strukturen befinden oder die Nukleinsäuren an die äußere Oberfläche einer Micelle, eines Liposoms oder einer ähnlichen nanopartikulären Struktur adsorbiert sind. Üblicherweise, ohne jedoch darauf beschränkt zu sein, enthalten die hier verwendeten Zusammensetzungen daher keine Micellen, Liposomen oder ähnliche nanopartikuläre Strukturen.

Die erfindungsgemäß zu verwendende Zusammensetzung kann beispielsweise bereitgestellt werden, indem Komplexe aus mindestens einer Nukleinsäure und mindestens einem organischen Kation in einem organischen Lösungsmittel oder einem Gemisch von organischen Lösungsmitteln gelöst werden und gegebenenfalls dann mit einer wässrigen Flüssigkeit gemischt werden. Zur Gewinnung der Komplexe kann zunächst die mindestens eine Nukleinsäure in einer wässrigen Flüssigkeit, insbesondere Wasser (z.B. demineralisiertes Wasser) gelöst werden, anschließend durch Zugabe von mindestens einem organischem Kation, das mit der Nukleinsäure einen in der wässrigen Lösung unlöslichen Komplex bildet, präzipitiert werden, und das Präzipitat ggf. gewaschen und, z.B. durch Trocknung, isoliert werden. Das Präzipitat ist vorzugsweise frei von jeglichen wässrigen Rückständen.

Die erfindungsgemäß zu verwendende Zusammensetzung kann daher zum Beispiel mit einem Verfahren hergestellt werden, dass folgende Schritte umfasst:
i) Bereitstellen einer Lösung mit mindestens einer in einer wässrigen ersten Flüssigkeit gelösten Nukleinsäure,
ii) Präzipitieren der mindestens einen Nukleinsäure durch Zusatz mindestens eines mit der Nukleinsäure in der ersten Flüssigkeit unlösliche Komplexe bildenden organischen Kations (Komplexbildner) zu der ersten Flüssigkeit,
iii) Abtrennen der Komplexe von der ersten Flüssigkeit, z.B. durch Trocknung,
iv) Lösen der Komplexe in mindestens einem organischen Lösungsmittel (zweite Flüssigkeit)
v) optional Mischen der zweiten Flüssigkeit mit einer dritten Flüssigkeit, z.B. einem Zellkulturmedium oder gepufferteren wässrigen Lösungen wie PBS, Tris etc.

Geeignete organische Kationen im Rahmen der vorliegenden Erfindung (bzw. Quellen für die Kationen) sind dabei beispielsweise kationische Detergenzien, Lipide und Ammoniumverbindungen, wie z.B. organische Ammoniumsalze. Zum Beispiel im Fall von Ammoniumverbindungen ist dann die mindestens eine Nukleinsäure in der Zusammensetzung derart komplexiert, dass die negativen Phosphatgruppen der Nukleinsäure durch Ammoniumgruppen der Ammoniumverbindung im Wesentlichen vollständig kompensiert sind.

Insbesondere können als Quelle für organische Kationen quartäre Ammoniumverbindungen verwendet werden. Dazu können eine oder mehrere Ammoniumverbindungen der Formel NR₄X verwendet werden, wobei jeder Rest R jeweils unabhängig voneinander ein verzweigter oder unverzweigter Kohlenwasserstoffrest mit 1 bis 20 Kohlenstoffatomen ist, und wobei X ein Halogenid-lon ausgewählt aus der Gruppe bestehend aus Chlorid, Bromid und lodid ist. Bevorzugt sind Chlorid oder Bromid.

Insbesondere können einer oder mehrere, insbesondere zwei oder drei, Reste R relativ kurzkettige Reste wie C1 bis C3, wie Methyl, Ethyl und Propyl sein, während ein Rest R ein längerer Rest wie C8 oder C10 bis C20, insbesondere C10 bis C16, wie Octyl, Nonyl, Decyl, Undecyl, Dodecyl, Tridecyl, Tetradecyl, Pentadecyl, Cetyl, Heptadecyl, Icosanyl, Stearyl oder Nonadecyl ist.

In einigen Ausführungsformen der Erfindung kann das mindestens eine organische Kation eine quartäre Ammoniumverbindung sein, wobei ein, zwei oder drei Reste ein C1 Rest sind, insbesondere Methyl, und entsprechend die anderen 3, 2 oder 1 Reste organische Reste darstellen, insbesondere längerkettige Reste mit z.B. mindestens 6 Kohlenstoffatomen. So können als organische Kationen Ammoniumionen vorliegen, bei denen zwei der vier Reste der quartären Ammoniumverbindung Methylgruppen sind und die anderen beiden organische Reste mit mindestens 6 Kohlenstoffatomen sind. Ein Beispiel hierfür sind Benzalkoniumionen und Dimethyldioctadecylammoniumionen. Andere Beispiele sind quartäre Ammoniumionen, bei denen drei der vier Reste Methylgruppen sind und der andere organische Rest mindestens 6 Kohlenstoffatome hat. Ein Beispiel hierfür sind Cetyltrimethylammoniumionen.

Vorzugsweise handelt es sich bei dem mindestens einen organischen Kation (bzw. der Quelle für das Kation) um ein oder mehrere quartäre Amine, wobei CTAB besonders bevorzugt ist. Andere Verbindungen, die sich erfindungsgemäß für die Komplexierung der Nukleinsäuren eignen, umfassen Benzethoniumchlorid, Benzalkonium, Benzalkoniumchlorid, Didecyldimethylammoniumbromid (DCAB), Dodecyltrimethylammoniumbromid (DCTAB), DOTAP, Lipofectin, Lipofectamin N-[1-(2,3-dioleyloxy)propyl]-N,N,N-trimethylammonium-chloride (DOTMA), Dimethyldioctadecyl-ammoniumbromid (DDAB), Dioleyldimethylammoniumchloride (DODAC), 2,3-Dioleoyloxy-N-[2-(spermidin carboxyamido)ethyl]-N-Ndimethyl-1-propaniniumtrifluoracetat (DOSPA), Diheptadecylamido-glycylspermidin (DHGS) und ähnliche. Vorzugsweise ist das organische Kation nicht Polylysin und/oder TC-Chol.

Vorzugsweise wird der Komplexbildner der ersten Flüssigkeit in Schritt ii) in gelöster Form zugesetzt. Dadurch wird eine schnellere Präzipitation ermöglicht als beim Zusatz eines ungelösten Komplexbildners. Darüber hinaus kann der Komplexbildner im, vorzugsweise leichten, Überschuss zugesetzt werden. Aber auch im Unterschuss kann der Komplexbildner bereits vereinzelt zu Präziptierung der Komplexe führen. Ziel ist es, dafür zu sorgen, dass die Nukleinsäure mit dem mindestens einen organischen Kation vorzugsweise so komplexiert wird, dass die negativen Ladungen der Nukleinsäuren durch positive Ladungen der organischen Kationen im Wesentlichen vollständig kompensiert sind, und insbesondere vorzugsweise ein Verhältnis von im Wesentlichen 1:1 zwischen den negativen Ladungen (d.h. den anionischen Gruppen) der mindestens einen Nukleinsäure und den Ladungen des mindestens einen organischen Kations vorliegt. Insbesondere organischen Kationen wie die oben erwähnten, z.B. Cetyltrimethylammoniumionen, sind ideal in der Lage, die positiv geladene Gruppe in einen ausreichend kleinen Abstand an das negativ geladene Sauerstoffatom der Nukleinsäure anzunähern, sodass eine ausreichende (lokale) Abschirmung der Gesamtladung beider Ionen bewirkt wird.

Vorteilhaft an der Einstellung eines solchen Verhältnisses ist, dass der Komplex aus organischen Kationen und Nukleinsäure insgesamt annähernd ladungsneutral ist, d.h. im Wesentlichen vollständig komplexiert ist. Die komplexierte Nukleinsäure ist daher in einem wässrigen Medium unlöslich und kann bei der Herstellung in wässrigen Medien als unlösliches Präzipitat abgetrennt werden. Diese Eigenschaft erleichtert somit die Herstellung der gewünschten, stöchiometrisch komplexierten Nukleinsäure. Das Abtrennen der Komplexe gemäß Schritt iii) erfolgt daher vorzugsweise mittels Zentrifugation oder Filtration und ggf. nachgeschalteten Wasch- und Trocknungsschritten. Diese Verfahren stellen eine besonders einfache und effiziente Art der Abtrennung der präzipitierten Komplexe dar. Das Präzipitat kann z.B. durch Zentrifugation zum Beispiel für 1 bis 10 Minuten, vorzugsweise 5 Minuten, bei 10000 bis 20000 g, vorzugsweise 15000 g, abgetrennt werden. Gegebenenfalls kann dieser Vorgang nach erneuter Aufnahme des gefällten Niederschlags in Wasser mehrmals durchgeführt werden. Vorteilhaft an der Einstellung eines solchen Verhältnisses ist ferner, dass beim In-Kontakt-Bringen der so komplexierten Nukleinsäure mit einer lebenden Zelle oder einem lebenden Zellgewebe (z.B. Organ) keine bzw. nur wenige freie organische Kationen mit den Zellen oder dem Zellgewebe wechselwirken können. Als Folge davon können die organischen Kationen kaum cytotoxischen Effekte oder andere Störungen des Metabolismus bei den Zellen auslösen, was ansonsten häufig vorkommen kann.

Im Zuge der Komplexierung der mindestes einen Nukleinsäuren können selbstverständlich wie oben erwähnt auch verschiedene organische Kationen Verwendung finden, indem man Mischungen unterschiedlicher organischer Kationen zu der Nukleinsäure-haltigen wässrigen Flüssigkeit gibt, um die Nukleinsäure-Komplexe zu präzipitieren.

Die durch Präzipitation erhaltenen Komplexe können, vorzugsweise nach Trocknung, anschließend in einer großen Vielzahl von organischen Lösungsmitteln (einschließlich solchen, die allgemein als Fällungsmittel für Nukleinsäure aus wässriger Lösung gelten) gelöst werden. Nach Aufnahme in ein Lösungsmittel enthalten diese im Wesentlichen vorzugsweise keine organischen Kationen, die über die zur Kompensation der negativen Ladungen der Nukleinsäure erforderliche Menge hinaus gehen. Dies bedeutet, dass die Anzahl der gesamten positiven Ladungen der organischen Kationen in dem Lösungsmittel im Wesentlichen mit der Anzahl der negativen Ladungen der Nukleinsäure identisch ist.

Organische Lösungsmittel, die zur Aufnahme der komplexierten Nukleinsäure geeignet sind, umfassen insbesondere ein- oder mehrwertige Alkohole oder teilweise veretherte Alkohole. Bevorzugt enthält das Lösungsmittel daher mindestens eine Ether-Gruppe und/oder mindestens eine Hydroxyl-Gruppe. Als Lösungsmittel besonders gut geeignete (amphiphile) Verbindungen können durch die Formel HO-R1-O-R2 beschrieben werden. Dabei ist R1 und R2 jeweils ein Kohlenwasserstoffrest mit 1 bis 100 Kohlenstoffatomen. Insbesondere sind C1 bis C5-Alkohole geeignet, wie z.B. Methanol, Ethanol, 1-Propanol oder 2-Propanol, Butanol, wie 1-Butanol, oder Pentadiol, wie 1-Pentadiol. Ferner sind mehrwertige Alkohole, wie Ethylenglykol oder teilweise veretherte Derivate davon, wie z.B. Ethylenglykol, das mit Methanol, Ethanol, Propanol oder Butanol verethert ist, wie beispielsweise Ethylenglykolether, wie insbesondere Ethylenglykolmonobutylether, -ethylether oder methylether, verwendbar. Auch Propylenglykol, Glycerin und Polyethylenglykole (PEG) können verwendet werden. Alternativ können gesättigte oder ungesättigte Kohlenwasserstoffe, wie Pentan, Hexan oder Heptan oder auf Benzol basierende aromatische Kohlenwasserstoffe, wie Styrol, verwendet werden, also insbesondere mit C1 bis C3-Resten substituierte Benzole. Alternativ können halogenhaltige Lösungsmittel wie Chloroform, Dichlormethan oder Tetrachlorkohlenstoff oder andere heteroatomhaltige Lösungsmittel, wie Dimethylformamid oder Tetrahydrofuran, verwendet werden. DMSO kann ebenfalls verwendet werden. Häufig wird für die erfindungsgemäß zu verwendenden Zusammensetzung ein organisches Lösungsmittel oder organisches Lösungsmittelgemisch gewählt werden, das mit Wasser homogen mischbar ist. Weitere organische Lösungsmittel, die auch in Frage kommen, insbesondere bei der Verwendung von Lösungsmittelgemischen, sind Öle, Wachse und Fette.

Insbesondere bei Verwendung von niederen Alkoholen wie Ethanol, Propanol oder Butanol als Lösungsmittel lassen sich Konzentrationen an komplexierter Nukleinsäure erreichen, die ansonsten nur in neutralen oder wässrigen Medien erreicht werden. So konnten bei Lösung von Heringssperma-DNA, die in einem ladungs-stöchiometrischen Verhältnis von etwa 1:1 mit CTAB komplexiert war, in den Lösungsmitteln Methanol, Ethanol, 1-Butanol, 2-Propanol, 1-Pentanol und Ethylenglykolmonobutylether jeweils eine Löslichkeit von mehr als 10 mg DNA pro ml Lösungsmittel erreicht werden.

In Lösungsmitteln, die alleine eine geringere Löslichkeit für die erfindungsgemäß komplexierten Nukleinsäuren zeigen, können ebenfalls Konzentration an gelöster Nukleinsäure von mehr als 1 mg/ml erreicht werden, indem die Nukleinsäure zunächst in einem ersten Lösungsmittel mit hoher Löslichkeit (wie z.B. Methanol, Ethanol, 1-Butanol, 2-Propanol, 1-Pentanol, Ethylenglykolmonobutylether oder Mischungen derselben) gelöst wird, und das die komplexierten Nukleinsäuren enthaltende erste Lösungsmittel anschließend mit einem zweiten Lösungsmittel homogen vermischt wird, das eine geringere Löslichkeit für die erfindungsgemäß komplexierten Nukleinsäuren aufweist. Geeignete Beispiele hierfür sind Öle, Wachse und Fette. In Walnussöl, Weizenkeimöl, Leinöl, Distelöl, Rapsöl und Mandelöl konnte so eine Konzentration von 100µg/ml) erreicht werden.

In bevorzugten Ausführungsformen der vorliegenden Erfindung umfassen die erfindungsgemäßen Zusammensetzungen daher mindestens ein Öl und/oder mindestens ein Wachs und/oder mindestens ein Fett.

Geeignete Öle, die zur Herstellung der erfindungsgemäßen Zusammensetzungen verwendet werden können, umfassen synthetische, tierische und pflanzliche Öle. Ebenso umfassen geeignete Wachse bzw. Fette synthetische, tierische und/oder pflanzliche Wachse bzw. synthetische, tierische und/oder pflanzliche Fette.

Geeignete pflanzliche Öle sind z.B. Nussöle und Samenöle. Geeignete pflanzliche Öle umfassen insbesondere Erdnussöl, Walnussöl, Mandelöl, Haselnussöl, Kokosnussöl, Sojabohnenöl, Olivenöl, Mohnöl, Hanföl, Kürbiskernöl, Sonnenblumensamenöl, Sesamsamenöl, Baumwollsamenöl, Distelöl, Leinöl, Rapsöl, Ricinusöl und Ähnliche. Auch aus Getreide gewonnene Keimöle können vorliegend verwendet werden. Bevorzugte Keimöle umfassen z.B. solche, die aus Mais, Weizen, Hafer, Roggen, Reis und Triticale gewonnen werden. Als geeignete pflanzliche Fette können beispielsweise Kokosfett oder Kakaobutter verwendet werden. Mögliche pflanzliche Wachse umfassen bspw. Zuckerrohrwachs, Carnaubawachs, Jojobaöl, Candelillawachs und Japanwachs.

Neben pflanzlichen Ölen, Wachsen und Fetten können auch tierische Fette, Wachse und Öle, wie z.B. Fischöle oder aus Nerzen oder Hirschen gewonnene Öle und Fette, eingesetzt werden. Lebertran und Walöl (wie beispielsweise aus Spermaceti) sind Beispiele für Fischöle, die vorliegend verwendet werden können. Mögliche Quellen für tierische Wachse sind beispielsweise Walrat, Wollwachs und Bienenwachs. Geeignete Verfahren zur Gewinnung reiner Öle tierischen Ursprungs sind im Stand der Technik bekannt.

Ggf. können auch synthetische Öle, Fette und Wachse eingesetzt werden. Geeignete synthetische Öle und Fette basieren zum Beispiel auf Silikon- oder Paraffinverbindungen. Ein Beispiel ist Vaseline. Sojawachs kann durch Hydrierung aus Soja gewonnen werden und ist ein Beispiel für ein synthetisches Wachs.

Wie bereits angedeutet umfassen die Zusammensetzungen der vorliegenden Erfindung wenn darin ein Öl, Wachs oder Fett vorliegt, bevorzugt mindestens ein weiteres Lösungsmittel, das kein Öl, Wachs oder Fett ist. Oder anders formuliert: besonders bevorzugt umfassen die erfindungsgemäß zu verwendenden Zusammensetzungen neben einem (konventionellen) organischen Lösungsmittel wie Ethanol, Ethylenglykolmonobutylether, Ethylenglykolmonoethylether, Ethylenglykolmonomethylether, und/oder DMSO etc. noch ein Öl, Fett oder Wachs. Eine besonders bevorzugte Kombination ist eine Kombination von DMSO mit mindestens einem Öl, Fett und/oder Wachs.

Die erfindungsgemäßen Zusammensetzungen können zum Beispiel einen Öl-, Wachs und/oder Fettanteil aufweisen, der größer oder gleich 25% (v/v), beispielsweise größer oder gleich etwa 30% (v/v), größer oder gleich etwa 40% (v/v), größer oder gleich etwa 50% (v/v), größer oder gleich etwa 60% (v/v), größer oder gleich etwa 70% (v/v), größer oder gleich etwa 80% (v/v), oder größer oder gleich etwa 85% (v/v) ist. In besonders bevorzugten Ausführungsformen weisen die erfindungsgemäß zu verwendenden Zusammensetzungen einen Öl-, Wachs und/oder Fettanteil von mehr als etwa 90%, vorzugsweise etwa 91% (v/v) oder mehr, etwa 92% (v/v) oder mehr, etwa 93% (v/v) oder mehr, etwa 94% (v/v) oder mehr, etwa 95% (v/v) oder mehr, etwa 96% (v/v) oder mehr, etwa 97% (v/v) oder mehr, etwa 98% (v/v) oder mehr, etwa 99% (v/v) oder mehr auf.

Für das erfindungsgemäße Verfahren sind die Nukleinsäurekomplexe besonders bevorzugt gelöst in einem organische Lösungsmittel ausgewählt aus der Gruppe bestehend aus:
a) Alkohol, wie Ethanol, 1-Butanol, 2-Propanol, 1-Pentanol,
b) DMSO, und
c) Glycolether wie z.B. Ethylenglykolmonobutylether.

Da wie bereits erwähnt die Nukleinsäurekomplexe auch in homogenen Mischungen von Lösungsmitteln gelöst vorliegen können, ist es ferner bevorzugt, wenn die Komplexe aus mindestens einer Nukleinsäure und mindestens einem organischen Kation in der erfindungsgemäß zu verwendenden Zusammensetzung in einem, vorzugsweise homogenen, Gemisch gelöst vorliegen ausgewählt aus der Gruppe bestehend aus:
a) zwei oder mehr Alkoholen,
b) einem oder mehr Alkoholen mit DMSO,
c) einem oder mehr Alkoholen mit einem oder mehr Glycolethern,
d) DMSO und einem oder mehr Glycolethern,
e) DMSO, einem oder mehr Alkoholen, und einem oder mehr Glycolethern,
f) einem Alkohol und einem Öl,
g) DMSO und einem Öl,
h) Glycolether und einem Öl
i) Gemischen von, vorzugsweise homogenen, Lösungsmittelgemischen gemäß a) bis e) mit einem oder mehr Ölen, oder
j) Gemischen von, vorzugsweise homogenen, Lösungsmittelgemischen gemäß a) bis i) mit einer wässrigen Flüssigkeit.

Die Gemische nach f) bis i) kommen insbesondere dann in Betracht, wenn Zellgewebe oder Organe mit der erfindungsgemäß zu verwendenden Zusammensetzung in Kontakt gebracht werden. Die Gemische nach j) kommen insbesondere dann in Betracht, wenn die ursprünglich in mindestens einem organischen Lösungsmittel gelösten Komplexe anschließend mit einer wässrigen Flüssigkeit, z.B. einem Zellkulturmedium oder, einer gepufferten Salzlösung, vermischt werden.

Die in organischen Lösungsmitteln gelösten Nukleinsäurekomplexe zeichnen sich durch eine außerordentlich hohe Stabilität aus. Dies ist im Wesentlichen darauf zurückzuführen, dass Nukleasen in nicht-wässriger Umgebung inaktiv sind. Dies ist vor allem bei Verwendung von Ribonukleinsäure (RNA) von Bedeutung, da in allen wässrigen Systemen mit einer hohen RNAse-Aktivität gerechnet werden muss. Außerhalb einer RNAse-freien Umgebung, die praktisch nur unter speziellen Laborbedingungen realisiert werden kann, ist grundsätzlich mit einem schnellen Abbau der RNA zu rechnen. Dieses Problem wird durch die Komplexierung der Nukleinsäure zu den vorliegend beschriebenen Komplexen gelöst.

Ferner ist die Stabilität der komplexierten Nukleinsäuren auch dadurch erhöht, dass in dem eingesetzten organischen Lösungsmittel vorzugsweise keine saure oder alkalische Umgebung mehr vorliegt. Dies vermeidet eine etwaige saure oder alkalische Hydrolyse der Nukleinsäuren.

Wie bereits erwähnt kann nach Lösung der Nukleinsäuren in dem mindestens einen organischen Lösungsmittel das organische Lösungsmittel beispielsweise mit beliebigen weiteren mit Wasser mischbaren Flüssigkeiten vermischt werden, z.B. mit einem weiteren der oben genannten organischen Lösungsmittel oder einer wässrigen Flüssigkeit. In den erfindungsgemäß zu verwendenden Zusammensetzungen kann das mindestens eine organische Lösungsmittel beispielsweise weniger als 70% (v/v), z.B. weniger als 50% (v/v), oder weniger als 20% (v/v) ausmachen. Das mindestens eine organische Lösungsmittel kann auch in einer Konzentration von mehr als 1 % (v/v), bevorzugt mehr als 5%(v/v), besonders bevorzugt mehr als 10% (v/v) vorliegen. So kann das organische Lösungsmittel beispielsweise in einem Bereich von 1-70% (v/v), 5-50% oder auch 10-20% (v/v) vorliegen. Die Konzentration des mindestens einen organischen Lösungsmittels in der Zusammensetzung sollte so gewählt werden, dass beim In-Kontakt-Bringen der Zusammensetzung mit den Zellen und/oder Zellgeweben kaum oder gar keine toxischen Auswirkungen des mindestens einen organischen Lösungsmittels auf die Zellen und oder Zellgewebe zu befürchten sind. Wird zum Beispiel ein großes Volumen der Zusammensetzungen einem kleinen Volumen an in Flüssigkeit suspendierter Zellen zugesetzt, oder ggf. sogar unmittelbar den Zellen zugesetzt, bei denen zuvor vollständig die umgebende Flüssigkeit abgenommen worden ist, so wird bevorzugt selbstverständlich eine geringe Konzentration des mindestens einen organischen Lösungsmittels in der Zusammensetzung gewählt werden. In Ausführungsformen, in denen ein kleines Volumen an Zusammensetzung einem verhältnismäßig größeren Volumen an in Flüssigkeit suspendierten Zellen zugesetzt wird, wird jedoch hingegen häufig eine hohe Konzentration des mindestens einen organischen Lösungsmittel tolerabel sein. Insbesondere in solchen Ausführungsformen (aber nicht darauf beschränkt) ist es dann besonders bevorzugt, wenn die Konzentration des organischen Lösungsmittels in der bereitgestellten Zusammensetzung , also vor dem In-Kontakt-Bringen mit den Zellen und/oder Zellgeweben, mindestens 80% (v/v), bevorzugt mindestens 90%, besonders bevorzugt mindestens 95% beträgt. Hohe Konzentrationen dieser Art, d.h. mindestens 80% (v/v), bevorzugt mindestens 90%, und besonders bevorzugt mindestens 95%, kommen beispielsweise auch in Betracht, wenn Zellgewebe oder Organe behandelt werden. Beispielsweise kann die entsprechende Stelle von Interesse des Organs ein entsprechend hochkonzentrierter Öltropfen aufgebracht werden.

Üblicherweise wird es sich bei der erfindungsgemäß zu verwendenden Zusammensetzung um eine Flüssigkeit handeln, schon weil in vielen Fällen das mindestens einen organische Lösungsmittel eine Flüssigkeit ist. Die hier beschriebenen Komplexierungsverfahren erlauben jedoch auch die Lösung entsprechend komplexierter Nukleinsäuren in bspw. Fetten. Zudem könnten die gelösten Nukleinsäuren verkapselt sein, wobei sich die Kapsel unter bestimmten Bedingungen oder zeitabhängig auflöst. Daher muss die Zusammensetzung nicht zwingend eine Flüssigkeit sein.

Die erfindungsgemäß zu verwendende Zusammensetzung kann, insbesondere wenn Sie in nahezu unverdünnter Form mit den Zellen und/oder Zellgeweben in Kontakt gebracht wird, bereits ein Zellkulturmedium umfassen. Unter einem "Zellkulturmedium" wird eine Flüssigkeit verstanden, die die Kultivierung der Zellen oder Zellgewebe erlaubt. Das Zellkulturmedium wird üblicherweise daher Nährstoffe wie essentielle Aminosäuren, Zucker, Salze und/oder Vitamine umfassen und/oder gepuffert sein. Die Auswahl des Zellkulturmediums wird vom Fachmann in Abhängigkeit der zu transfizierenden Zellen ausgewählt werden und üblicherweise dasjenige sein, in denen die Zellen bereits vor der Anwendung des vorliegenden Verfahrens kultiviert worden sind.

Die erfindungsgemäß in die Zellen oder Gewebe einzubringende Nukleinsäure ist eine geladene Nukleinsäure. Der Begriff "Nukleinsäure" ist erfindungsgemäß eine Nukleinsäure zu verstehen, deren Nukleoside durch Phosphodiesterbindungen miteinander verknüpft sind, wobei die an den Phosphodiesterbindungen beteiligten Phosphatreste negativ geladen sind, wie es bei natürlich vorkommender DNA oder RNA der Fall ist. Mit anderen Worten handelt es sich bei den geladenen Nukleinsäuren um polyanionische Säuren (Polyelektrolyte), in denen die anionischen Gruppen durch die negativ geladenen Sauerstoffreste der Phosphatgruppen, die die Phosphordiesterbindungen bilden, gebildet werden. Grundsätzlich kann es sich bei den die negativen Ladungen tragenden Resten der Nukleinsäuren auch um die negativen Reste von Phosphorthioaten oder Phosphordithioaten oder auch um andere negativ geladene Gruppen von Nukleinsäuren handeln.

Üblicherweise lösen sich Nukleinsäuren auf Grund ihrer Ladung gut in Wasser, nicht jedoch in mit Wasser nicht mischbaren organischen Flüssigkeiten, wie Kohlenwasserstoffen oder anderen organischen Lösungsmitteln, wie Chloroform, Ölen etc. Eine Nukleinsäure wird im Sinne der Erfindung als gelöst erachtet, wenn sie sich durch eine 5-minütige Zentrifugation bei 15000 x g nicht abzentrifugieren lässt.

Bei den Nukleinsäuren kann es sich um einzelsträngige oder doppelsträngige Nukleinsäuren handeln, wie z.B. um einzelsträngige oder doppelsträngige DNA oder RNA. Eine doppelsträngige Nukleinsäure kann bspw. in Form zweier separater Einzelstränge oder als Haarnadeischleifenstruktur vorliegen. Die Größe der einzubringenden Nukleinsäure ist erfindungsgemäß unkritisch. Es konnte gezeigt werden, dass sowohl Oligonukleotide mit einer Kettenlänge von 10 bis 100 Nukleotiden, als auch lineare und zirkuläre Plasmide mit einer Größe von mehreren kb (z.B. mit einer Größe von 1-10 kb) wirksam in humane und murine Hautzellen eingebracht werden können. Damit eröffnet das Verfahren der vorliegenden Erfindung neue Möglichkeiten sowohl auf dem Gebiet der Genmanipulation, bei der z.B. Nukleinsäuren, welche für bestimmte Polypeptide kodieren, in Zellen eingebracht werden, als auch auf dem Gebiet der RNA-Interferenz, bei der zielgerichtet die Expression bestimmter Gene beeinflusst wird.

In einer besonderen Ausführungsform der Erfindung handelt es sich bei der Nukleinsäure um ein Oligonukleotid, d.h. um einzelsträngige oder doppelsträngige DNA oder RNA (oder DNA/RNA Hybride) mit einer Kettenlänge von etwa 5 bis etwa 150 Nukleotiden, bevorzugt von etwa 7 bis etwa 100 Nukleotiden, bevorzugt etwa 10 bis etwa 80 Nukleotiden, besonders bevorzugt etwa 15 bis etwa 60 Nukleotiden. Das Oligonukleotid ist vorzugsweise ein synthetisch hergestelltes Oligonukleotid mit bekannter Sequenz.

Bei den Nukleinsäuren kann es sich beispielsweise um DNA, cDNA, mRNA, sRNA, Ribozyme, Antisense-DNA, RNA, DNA/RNA-Hybride, Antisense RNA, siRNA, Decoys, tRNA, rRNA, snRNA, snoRNA, und/oder miRNA handeln. In einer besonderen Ausführungsform ist die Nukleinsäure keine siRNA. Für einige Anwendungen ist es besonders vorteilhaft, wenn die Nukleinsäuren zumindest einen Teil einer Sequenz eines (humanen) Genoms oder auch Gens enthalten.

In einer besonderen Ausführungsform der Erfindung handelt es sich bei der Nukleinsäure um einen Vektor oder um ein Plasmid mit einer Größe von mehr als 1 kb (1000 Nukleotide). Die Größe des Vektors oder des Plasmids wird in der Regel mehr als 2 kb, 3 kb, 4 kb, 5 kb, 6 kb, 7 kb, 8 kb, 9 kb, 10 kb, 11 kb, 12 kb, 13 kb, 14 kb, 15 kb oder sogar mehr als 20 kb betragen. Es kann sich bei der Nukleinsäure, die in die Zellen eingebracht werden soll, z.B. um einen Expressionsvektor handeln, der für die Expression mindestens eines Gens in die Zellen eingebracht wird. Beispiele für Expressionsvektoren, die für das erfindungsgemäße Verfahren eignen, umfassen virale und nicht-virale Vektoren. Virale Vektoren leiten sich von verschiedenen Viren ab, z.B. von Retroviren, Herpesviren, Adenoviren oder von Adenoassoziierten Viren (AAV), siehe Lundstrom, Trends Biotechnol. (2003), 21 (3):117-22. Vorzugsweise handelt es sich bei den viralen Vektoren um solche, die nicht mehr in der Lage sind, sich in den damit transfizierten Zellen zu replizieren. Neben den viralen Vektoren können auch nicht-virale Vektoren, z.B. eukaryontische Expressionsvektoren, wie vorliegend beschrieben, in die Zellen oder Gewebe eingebracht werden. Die Vektoren oder Plasmide umfassen vorzugsweise einen Promotor, der in den Zellen aktiv ist.

Falls gewünscht kann die Nukleinsäure eine nachweisbare Markierung, z.B. eine Fluoreszenzmarkierung umfassen, um eine erfolgreiche Einbringung in Zellen und Zellgewebe nachweisen zu können.

Vorzugsweise ist die erfindungsgemäß zu verwendende Nukleinsäure kein Oligonukleotid mit der Sequenz (3'-GAT CCT GCA TAT GGT AGT G -5' (SEQ ID NO: 1), insbesondere kein mit TAMRA oder 6FAM markiertes Oligonukleotid mit der Sequenz (3'-GAT CCT GCA TAT GGT AGT G -5' (SEQ ID NO: 1).

Die oben beschriebene Zusammensetzung wird im erfindungsgemäßen Verfahren mit den Zellen und/oder Zellgeweben ex vivo in Kontakt gebracht.

Dieses In-Kontakt-Bringen kann auf unterschiedlichste Weise erfolgen. In bevorzugten Ausführungsformen wird hierfür eine flüssige Zusammensetzung wie oben beschrieben verwendet und mit in (vorzugsweise wässriger) Flüssigkeit suspendiert vorliegenden Zellen und/oder Zellgeweben vermischt. Daher wird insbesondere das (mindestens eine) organische Lösungsmittel vorzugsweise so gewählt, dass es mit Wasser homogen vermischbar ist. Beim Mischen wird in Abhängigkeit der Volumenverhältnisse der Zusammensetzung einerseits und der die Zellen bzw. Zellgewebe enthaltenden Flüssigkeit die Konzentration des organischen Lösungsmittels und der darin gelösten Nukleinsäurekomplexe verringert. Daher sollte vorzugsweise je kleiner das Verhältnis von erfindungsgemäß zu verwendender Zusammensetzung zur zellhaltigen Flüssigkeit ist, eine desto höhere Konzentration des (mindestens einen) organischen Lösungsmittels mit den darin gelösten Nukleinsäurekomplexen in der Zusammensetzung gewählt werden.

Ebenso denkbar ist, dass die Zellen und/oder Zellgewebe nahezu ohne umgebende Flüssigkeit vorliegen und nur noch von einem Flüssigkeitsfilm benetzt sind. Dies ist beispielsweise der Fall wenn in Suspension wachsende Zellen abzentrifugiert werden und der flüssige Überstand abgenommen wird. In diesem Fall können die Zellen, bzw. Zellgewebe unmittelbar durch Resuspendierung in den oben beschriebenen Zusammensetzungen mit den Nukleinsäurekomplexen in Kontakt gebracht werden. Gleiches gilt für adhärente Zellen, bei denen ebenfalls der flüssige Überstand abgenommen werden kann. Hier kann die Zusammensetzung unmittelbar auf die Zellen aufgebracht werden. Daher umfasst die Erfindung auch Verfahren, bei denen die oben beschriebenen Zusammensetzungen eine Flüssigkeit ist, und wobei das In-Kontakt-Bringen das Aufbringen der flüssigen Zusammensetzung auf eine mit Zellen und/oder Zellgewebe bedeckte Oberfläche (z.B. der Boden einer Zellkulturschale oder - flasche oder eines Lochs einer Zellkulturplatte, der spitze Boden eines Microreaktionsgefäßes wie sie von der Firma Eppendorf erhältlich sind, etc.). Hierunter fallen auch Ausführungsformen, bei denen größere Zellgewebe, ggf. sogar Organe, ex vivo behandelt werden sollen. In solchen Ausführungsformen kann die entsprechende Zusammensetzung auch auf das entsprechende Zellgewebe bzw. Organ an der Stelle von Interesse aufgebracht werden. Ebenso ist denkbar, dass das gesamte Zellgewebe bzw. Organ in die erfindungsgemäßen Zusammensetzung einfach eingebettet bzw. eingetaucht wird. In all diesen Fällen, bei denen die Zusammensetzung nahezu unverdünnt den Zellen zugesetzt wird, verringert sich die die Konzentration des mindestens einen organischen Lösungsmittels und der darin gelösten Nukleinsäurekomplexe selbstverständlich kaum.

Betrifft das erfindungsgemäße Verfahren intakte Zellgewebe oder gar Organe, umfasst die erfindungsgemäß zu verwendende Zusammensetzung vorzugsweise eine Öl, in dem die Komplexe gelöst sind. Die intakten Zellgewebe und Organe sollten in solchen Ausführungsformen auch vorzugsweise gekühlt werden.

Der Fachmann wird daher je nach Art der Durchführung des In-Kontakt-Bringens die entsprechende Konzentration des (mindestens einen) organischen Lösungsmittels in der Zusammensetzung wählen, um während des In-Kontakt-Bringens eine weitestgehend zellverträgliche Konzentration an organischem Lösungsmittel zu gewährleisten. In bevorzugten Ausführungsformen wird während des In-Kontakt-Bringen der Zusammensetzung mit den Zellen und/oder Zellgewebe die Konzentration des mindestens einen organischen Lösungsmittels, in dem die Komplexe aus mindestens einer Nukleinsäure und mindestens einem organischen Kation gelöst vorliegen, weniger als 10% (v/v), noch bevorzugter weniger als 1 %, besonders bevorzugt weniger als 0,2% betragen.

Das In-Kontakt-Bringen kann eine längere Inkubation umfassen. Die Dauer des Kontakts zwischen der erfindungsgemäßen Zusammensetzung und den Zellen bzw. Zellgeweben kann von mehreren Faktoren beeinflusst werden. Grundsätzlich gilt, dass je toxischer die gewählte Konzentration des organischen Lösungsmittel ist, desto kürzer sollte die Exposition sein. In der Regel wird in Abhängigkeit von der Art der Zellen oder Zellgeweben, in die die komplexierten Nukleinsäuren eingebracht werden soll, sowie in Abhängigkeit von der Größe und Menge der einzubringenden Nukleinsäure die Exposition im Bereich von Minuten oder Stunden liegen. Bei einigen Anwendungen kann es allerdings auch vorteilhaft sein, eine Verweildauer von mehreren Tagen vorzusehen, insbesondere wenn das Einbringen einer großen Menge Nukleinsäure in die Zellen oder Gewebe erwünscht ist. Dies ist möglich weil die vorliegende Erfindung auch das In-Kontakt-Bringen in Gegenwart von Zellkulturmedium, Serum (z.B. fötales Kälberserum), Proteinen und/oder Antibiotika erlaubt. Die Inkubation kann daher über mehrere Minuten, z.B. mehr als 15 Minuten, erfolgen, oder auch für Stunden, zum Beispiel über Nacht, oder gar über Tage hinweg.

Das erfindungsgemäße ex vivo Verfahren eignet sich zur Einbringung von Nukleinsäuren in (isolierte) Zellen oder (isolierte) Zellgewebe. Vorzugsweise handelt es sich bei den Zellen oder Zellgeweben, die durch die Einbringung der Nukleinsäurekomplexe behandelt werden sollen, um Zellen oder Zellgewebe eukaryotischen, insbesondere tierischen Ursprungs, z.B. Zellen oder Zellgewebe von Säugetieren. Beispiele hierfür sind Zellen und/oder Zellgewebe von Affe, Maus, Ratte, Hamster, Meerschweinchen, Hund, Katze, Pferd, Rind, Schwein, Schaf, Ziege und anderen. In einer besonders bevorzugten Ausführungsform handelt es sich um menschliche Zellen und/oder Zellgewebe. Soweit es sich um menschliche Zellen handelt, sind diese vorzugsweise keine embryonalen Stammzellen.

Häufig wird es sich bei den zu behandelnden Zellen um Zellen handeln, die in Zellkultur gehalten werden können. Die Zellen können beispielsweise Tumorzellen oder auf anderem Wege immortalisierte Zellen sein. Es kann sich um adhärent wachsende Zellen oder nichtadhärente, z.B. in Suspension wachsende Zellen handeln. Selbstverständlich kann es sich bei den Zellen auch um Primärzellen handeln, die einem Organismus zu einem früheren Zeitpunkt entnommen worden sind.

Schließlich sei nochmals darauf hingewiesen, dass das erfindungsgemäße Verfahren ohne die Nutzung von Liposomen auskommt, d.h. die beschriebenen Komplexe liegen üblicherweise nicht innerhalb von Liposomen oder an die Oberfläche von Liposomen gebunden vor.

### Kurze Beschreibung der Figuren

- Fig. 1: zeigt den Dünnschnitt humaner Hautfragmente nach Behandlung mit fluoreszierenden Oligonukleotiden aus Beispiel 3. In Fig. 1 sind die Zellkerne durch Färbung mit Bisbenzimid dargestellt und waren im blauen Kanal des Fluoreszenzmikroskops (helle Bereiche) sichtbar.
- Fig. 2: zeigt den gleichen Schnitt wie Figur 1, wobei der grüne Kanal dargestellt ist. Die grüne Fluoreszenz (helle Bereiche) zeigen die Fluoreszenz des aufgetragenen FAM-Oligonukleotids an. Die Fluoreszenz ist auf der Oberfläche der Haut (rechter Rand) in den darunterliegenden Zellen zu beobachten. Die hellen Bereiche hinter der Hautoberfläche decken sich mit den Bereichen der Zellkerne in Figur 1.
- Fig. 3: zeigt Fluoreszenzaufnahmen (Fig. 3a und 3b) von C2C12-Mausmyoblasten, die mit Cetyltrimethylammonium-komplexierte einzelsträngige DNA aufgenommen haben. Die DNA-Komplexe waren zuvor, in Ethanol gelöst, mit den Zellen in Kontakt gebracht worden.
- Fig. 4: zeigt Fluoreszenzaufnahmen von C2C12-Mausmyoblasten, die als Kontrolle dienten und denen keine Cetyltrimethylammonium-komplexierte einzelsträngige DNA zugesetzt worden war.
- Fig. 5: zeigt Fluoreszenzaufnahmen von C2C12-Mausmyoblasten, die mit Cetyltrimethylammonium-komplexierte doppelsträngige DNA aufgenommen haben. Die DNA-Komplexe waren zuvor, in DMSO gelöst, mit den Zellen in Kontakt gebracht worden.

Die folgenden Ausführungsbeispiele veranschaulichen weitere bevorzugte Ausführungsformen der vorliegenden Erfindung, wobei die vorliegende Erfindung nicht auf diese spezifischen Ausführungsbeispiele beschränkt ist.

### Beispiel 1

### Herstellung von Oligonukleotiden

Folgende Oligonukleotide wurden für die transdermale Verabreichung an menschliche Haut bzw. Mäusehaut vorgesehen.

Ein Oligonukleotid (3'-GAT CCT GCA TAT GGT AGT G -5' (SEQ ID NO: 1), Molekulargewicht 5938,82g/Mol ) war mit dem fluoreszenten Farbstoff TAMRA markiert (Oligonukleotid 1). Oligonukleotid 2 (Molekulargewicht 5938,82g/Mol ) war mit dem fluoreszenten Farbstoff Fluorescein 6FAM markiert, um einen Nachweis des Oligonukleotids in den Hautzellen zu ermöglichen.

Die Oligonukleotide wurden von der Firma Biolegio (6545 CG Nijmegen, Niederlande) bezogen.

In weiteren Experimenten wurd DNA-Oligonukleotid 3 (5'- GAG GCG ATG TCT CTC ATG AT - 3; SEQ ID NO: 2) verwendet und mit dem fluoreszenten Farbstoff Fluorescein (6FAM) am 5'-Ende markiert, um ebenfalls einen Nachweis des Oligonukleotids in den Hautzellen zu ermöglichen. Dieses Oligonukleotid wurde von der Firma Thermo Electron GmbH (Ulm, Deutschland) bezogen.

### Beispiel 2

Zur Herstellung von CTAB-Oligonukleotid-Komplexen wurde 1 mg der Oligonukleotide in 200 µl Wasser gelöst und mit 400 µl 10mM CTAB versetzt. Die Probe wurde 30 min auf Eis inkubiert und der entstandene Niederschlag 5 min bei 10000 xg zentrifugiert. Der Überstand wurde abgezogen und der Niederschlag mit 200 µl Wasser suspendiert und erneut zentrifugiert. Der Niederschlag wurde über Nacht im Speedvac getrocknet.

### Beispiel 3

### Einbringen von DNA in humane Hautzellen

Die in Beispiel 2 hergestellten CTAB-Nukleinsäure-Komplexe wurden nach dem Trocknen gewogen und in dem organische Lösungsmittel Ethylenglycolmonoethylether (EGE) bzw. Butanol aufgenommen, wobei die Endkonzentration der komplexierten Nukleinsäure etwa 1mg/ml betrug. Die in Ethylenglycolmonoethylether gelösten CTAB-Nukleinsäure-Komplexe wurden anschließend mit Mandelöl in einem Volumenverhältnis von Öl:Ethylenglycolmonoethylether von 9:1 vermischt (90% (v/v) Öl, 10% (v/v) EGE). Die Endkonzentration der komplexierten Nukleinsäure in der jeweiligen Mischung betrug somit etwa 100 µg/ml.

Jeweils 5 µl der obigen Mischung aus Öl und EGE bzw. Butanol wurden auf Stücke aus Vliespapier (2 mm x 2 mm, Whatman) aufgebracht, die eine Dicke von 1 mm aufwiesen. Die mit dem Nukleinsäure-haltigen Lösungsmittel getränkten Vliespapierstücke wurden auf humane Hautfragmente (ca. 1 cm x 4 cm) aufgebracht, welche aus einer frischen Bauchoperation erhalten wurden. Die Papierstücke wurden getrennt voneinander in verschiedenen Bereichen des Hautfragments mit Klebeband fixiert. Als Kontrolle wurde eine Mischung von 90% (v/v) Öl, 10% (v/v) EGE, bzw. Butanol verwendet.

Das entsprechend vorbereitete Hautfragment wurde in eine Petrischale mit 5 ml Ringerlösung überführt und über Nacht bei 37°C im CO₂-Inkubator inkubiert. Anschließend wurde das Hautfragment vorsichtig mit einem Skalpell zerteilt, so dass Teilfragmente erhalten wurden, die jeweils ein Vliespapier auf ihrer Oberflächen aufwiesen. Die Teilfragmente wurden für die weitere Lagerung in flüssigen Stickstoff eingefroren. Die eingefrorenen Teilfragmente wurden für die Herstellung von Dünnschnitten mit einer Dicke von etwa 8 µm verwendet. Es wurde eine Kernfärbung mit Bisbenzimid (5 min, 1 µg/ml Bisbenzimid in PBS) durchgeführt. Die Auswertung erfolgte durch Fluoreszenzmikroskopie.

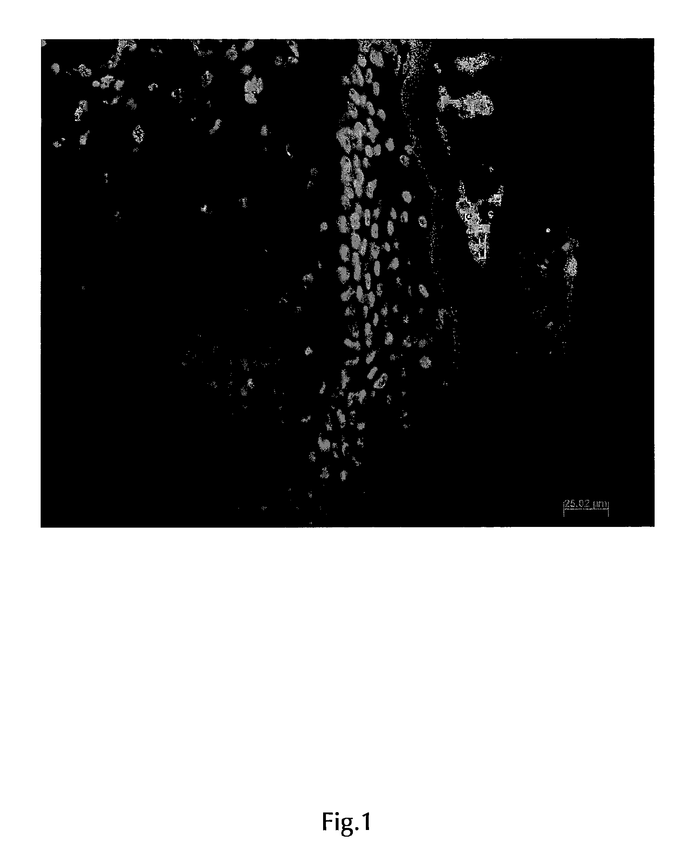

Die Ergebnisse der Fluoreszenzmikroskopie sind in den Figuren 1-2 dargestellt. Die von den markierten Oligonukleotiden emittierte Fluoreszenz konnte in den Epithelzellen und darüber hinaus auch in den darunter liegenden Bindegewebszellen nachgewiesen werden. Auch die Zellkerne zeigten eine Fluoreszenz, was darauf verweist, dass die markierten Oligonukleotide in die Zellkerne der Hautzellen vordringen konnten.

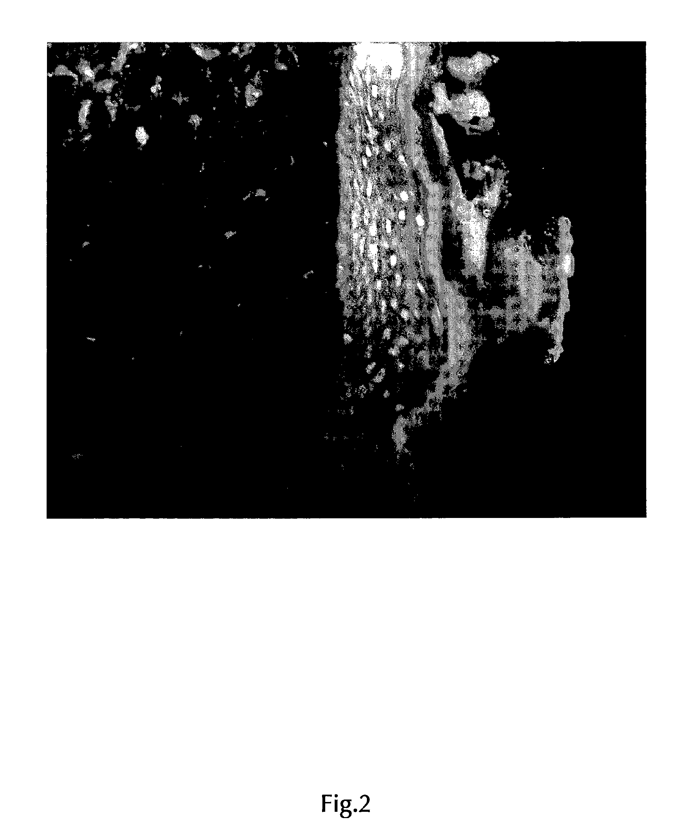

Figur 1 zeigt eine fluoreszenzmikroskopische Aufnahme eines Dünnschnitts eines Hautfragments, das wie oben beschrieben behandelt wurde. Deutlich sind die mittels Bisbenzimid-Färbung sichtbar gemachten Zellkerne zu erkennen, die als hell gefärbte Bereiche auftreten. Figur 2 zeigt den gleichen Dünnschnitt wie Figur 1, bei der die Fluoreszenz des FAM-Oligos gezeigt ist. Die hellen Bereiche zeigen das fluoreszierende Oligonukleotid. Es ist zu erkennen, dass die hellen Bereiche sich mit den hellen Bereichen aus Figur 1 decken. Diese Übereinstimmung zeigt, dass die fluoreszierenden Oligonukleotide in die Zellkerne der Hautzellen eindringen konnten.

Hautfragmente, die mit TAMRA-markierten Oligonukleotid behandelt worden waren, zeigten eine entsprechende rote Fluoreszenz der Zellkerne. Hautfragmente, die lediglich mit der Kontrolle (ohne Oligonukleotid) behandelt worden waren, zeigten keine Fluoreszenz der Zellkerne.

Ähnliche Ergebnisse wurden mit Oligonukleotid 3 erhalten.

### Beispiel 4

### Herstellung von fluoreszierenden, Cetyltrimethylammonium-komplexierter einzelsträngiger Nukleinsäure in Ethanol

1 mg einer fluoreszierenden, synthetischen DNA (FAM-DNA, Sequenz-Nr. 1, Hersteller Firma Biolegio B.V., PO BOX 91, 6500 AB Nijmegen, Niederlande) wurde in 500 µl Wasser gelöst. Zu der entstandenen Lösung wurde ein gleiches Volumen 100 mM wässriger Lösung von Cetyltrimethylammoniumbromid gegeben und die Flüssigkeit gemischt. Es entstand ein gelber Niederschlag, der durch 5 min Zentrifugation bei 15.000xg abzentrifugiert wurde. Die flüssige Oberphase mit dem überschüssigen Cetyltrimethylammoniumbromid wurde verworfen und der Niederschlag in 1 ml demineralisiertem Wasser suspendiert und erneut wie oben zentrifugiert. Die wässrige Phase wurde verworfen und der Niederschlag über Nacht im Vakuum getrocknet. Der getrocknete Niederschlag wurde in 100µl Ethanol gelöst.

### Beispiel 5

### Herstellung von fluoreszierender, Benzalkonium-komplexierter doppelsträngiger Nukleinsäure in Dimethylsulfoxid

10 mg Heringssperma DNA (HS-DNA, Serva, Deutschland) wurde in 1 ml Wasser gelöst. Zu der entstandenen Lösung wurde 500 µl 200 mM wässriger Benzalkoniumchlorid-Lösung (Sigma, Deutschland) und 10µl 1µg/µl Ethidiumbromid-Lösung in Dimethylsulfoxid gegeben und die Flüssigkeit gemischt. Es entstand eine rötliche Trübung, die durch 5 min Zentrifugation abzentrifugiert wurde. Die flüssige Oberphase mit dem überschüssigen Benzalkoniumchlorid wurde verworfen und der Niederschlag in 1 ml demineralisiertem Wasser suspendiert und erneut wie oben zentrifugiert. Die wässrige Phase wurde verworfen und der Niederschlag über Nacht im Vakuum getrocknet. Der getrocknete Niederschlag wurde in 2 ml Dimethylsulfoxid gelöst.

### Beispiel 6

### Aufnahme von fluoreszierender Cetyltrimethylammonium-komplexierter einzelsträngige Nukleinsäure in Zellkulturzellen

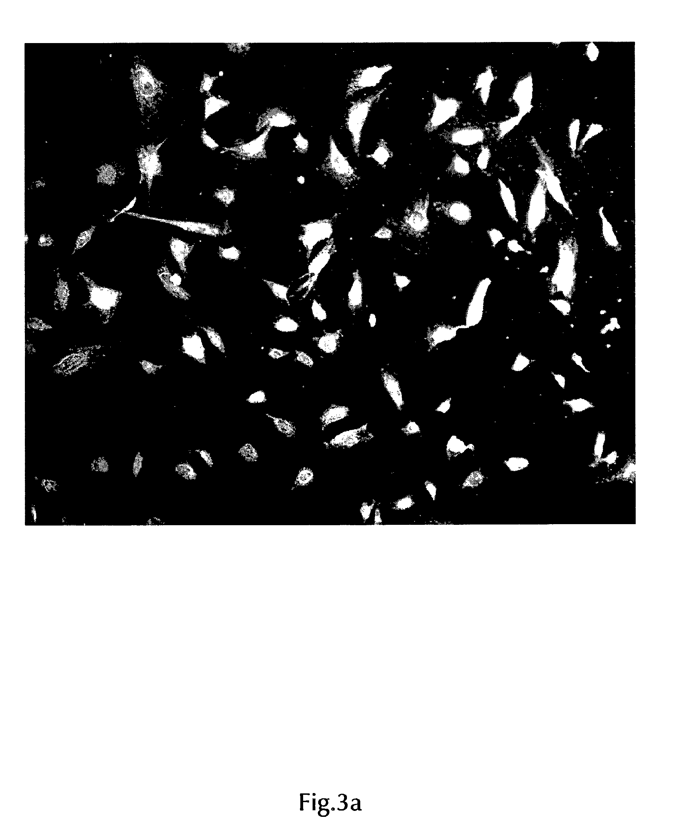

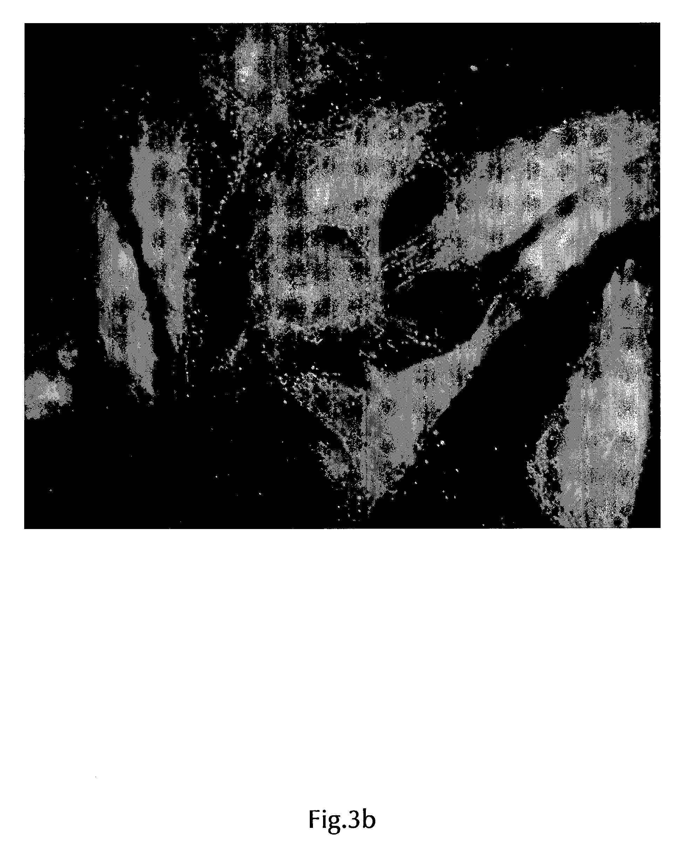

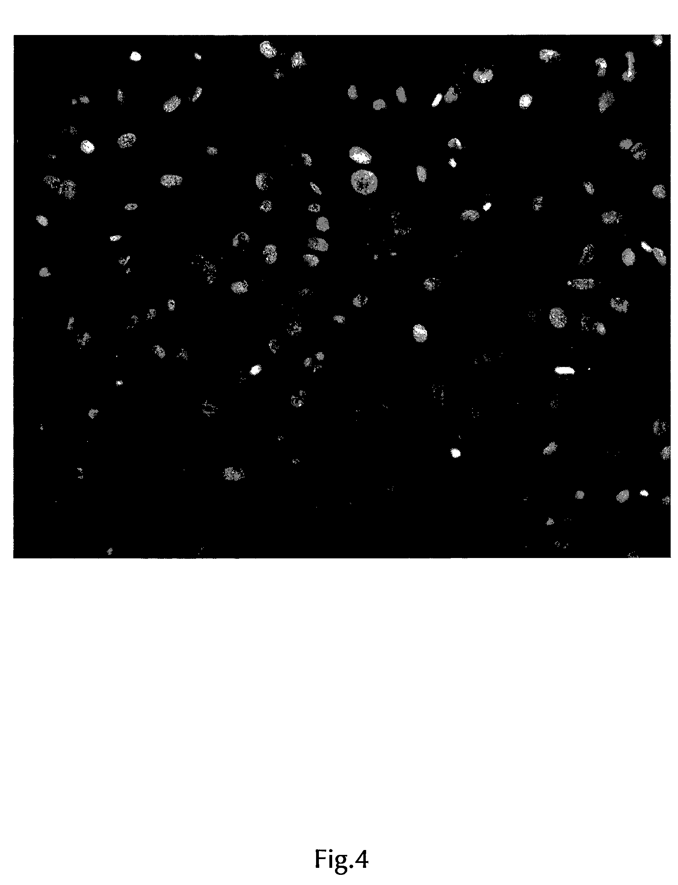

In eine 6-Loch Zellkulturschale wurden je Loch ein steriles, rundes Deckglas (Durchmesser 1 cm) gelegt. Ca. 200.000 Zellen frisch trypsinierter C2C12-Mausmyoblasten wurden in 4 ml F10-Medium (Life Technologies GmbH, Darmstadt, Deutschland) mit 20% (v/v) (Life Technologies GmbH, Darmstadt, Deutschland) fötalem Kälberserum in jedes Loch eingefüllt. Nach ca. vier Stunden im CO2-Inkubator (37°C, 5% (v/v) CO2) hatten sich die Zellen auf den Boden der Löcher adhäriert. In die Löcher 1-5 wurde zu den Zellen 0 µl, 1 µl, 2,5 µl, 5 µl und 10 µl Cetyltrimethylammonium-komplexierter einzelsträngiger Nukleinsäure aus Beispiel 4 pipettiert. In Loch 6 wurde zur Kontrolle 10 µl 2 µg/µl FAM-DNA in PBS gegeben. Die Zellen wurden über Nacht im CO2-Inkubator (37°C, 5% (v/v) CO2) inkubiert. Das Wachstum der Zellen wurde mittels Invertmikroskop untersucht. Das Wachstum der Zellen war in allen Löchern außer Loch 5 (10 µl Nukleinsäurelösung) im Wesentlichen gleichförmig. Zur Kernfärbung wurden das Medium abgezogen und die Zellen mit je 1 ml 2 µg/ml Dapi (Sigma, Taufkirchen, Deutschland) in PBS (phosphate buffered saline) überschichtet. Nach 15min wurde die Dapi-Lösung entfernt und die Zellen mit einem 1 ml PBS gewaschen. Die Deckgläser wurden aus den Löchern entnommen und mit einem Tropfen Fluoromount (Sigma, Taufkirchen, Deutschland) auf Objektträger überführt und im Fluoreszenzmikroskop fotografiert. Die mit Cetyltrimethylammonium-komplexierter Nukleinsäure versetzten Zellen zeigten im Fluoreszenzmikroskop eine grüne Fluoreszenz (siehe Figur 3a und 3b). Zellen ohne Zugabe von komplexierter Nukleinsäure (Loch 6) zeigten keine Fluoreszenz im Zytosol (Figur 4).

### Beispiel 7

### Aufnahme von fluoreszierender Cetyltrimethylammonium-komplexierter einzelsträngiger Nukleinsäure in Zellkulturzellen mit Langzeit-gelagerter Nukleinsäurelösung

Es wurde das gleiche Experiment durchgeführt wie in Beispiel 6 beschrieben, wobei die verwendete Ethanollösung mit der komplexierten Nukleinsäure vor der Verwendung 2 Monate bei Raumtemperatur in einem Kunststoff-Röhrchen gelagert worden war. Das Ergebnis war das gleiche wie in Beispiel 6. D.h. die Zellen, die mit der komplexierten Nukleinsäurelösung behandelt worden waren, zeigten eine grüne Fluoreszenz, während die Kontrollen ohne Nukleinsäure (Loch 1 und Loch 6) keine grüne Fluoreszenz zeigten.

### Beispiel 8

### Aufnahme von fluoreszierender, doppelsträngiger komplexierter Nukleinsäure in Zellkulturzellen

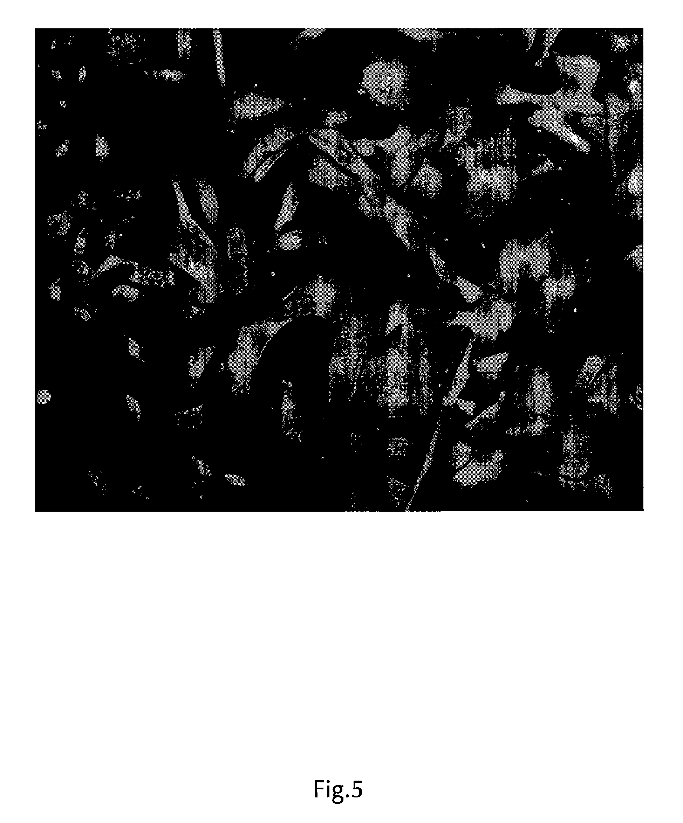

In eine 6-Loch Zellkulturschale wurden je Loch ein steriles, rundes Deckglas (Durchmesser 1 cm) gelegt. Ca. 200.000 Zellen frisch trypsinierter C2C12-Mausmyoblasten wurden in 2ml F10-Medium mit 20% (v/v) fötalem Kälberserum in jedes Loch eingefüllt. Nach ca. drei Stunden im CO₂-Inkubator (37°C, 5% (v/v) CO₂) hatten sich die Zellen auf den Boden der Löcher adhäriert. Zu den Zellen wurde in die Löcher 1-5 0 µl, 1 µl, 2,5 µl, 5 µl und 10 µl Benzalkonium-komplexierter Nukleinsäure aus Beispiel 5 pipettiert. In Loch 6 wurde zur Kontrolle 10 µl 1µg/µl) HS-DNA mit 1 ng/µl Ethidiumbromid in PBS gegeben. Die Zellen wurden vier Stunden im CO₂-Inkubator (37°C, 5% (v/v) CO₂) inkubiert. Danach wurde das Medium abgesaugt und durch frisches Medium ohne komplexierter Nukleinsäure ersetzt. Die Zellen wurden über Nacht im CO₂-Inkubator (37°C, 5% (v/v) CO₂) inkubiert. Das Wachstum der Zellen wurde mittels Invertmikroskop untersucht. Das Wachstum der Zellen war in allen Löchern im Wesentlichen gleichförmig. Zur Kernfärbung wurden das Medium abgezogen und die Zellen mit je 1 ml 2 µg/ml Dapi in PBS überschichtet. Nach 15 min wurde die Dapi-Lösung entfernt und die Zellen mit einem 1 ml PBS gewaschen. Die Deckgläser wurden aus den Löchern entnommen und mit einem Tropfen Fluoromount auf Objektträger überführt und im Fluoreszenzmikroskop fotografiert. Die mit Benzalkonium-komplexierter Nukleinsäure versetzten Zellen zeigten im Fluoreszenzmikroskop eine rote Fluoreszenz (siehe Figur 5). Mit nicht-komplexierter DNA behandelte Zellen, zeigten keine Aufnahme der DNA.

## Patentansprüche

1. Ex vivo Verfahren zur Einbringung von Nukleinsäuren in Zellen und/oder Zellgewebe, wobei das Verfahren folgende Schritte umfasst:
a) Bereitstellen einer Zusammensetzung, die mindestens ein organisches Lösungsmittel umfasst, in dem Komplexe aus mindestens einer Nukleinsäure und mindestens einem organischen Kation gelöst vorliegen, und
b) In-Kontakt-Bringen der Zusammensetzung nach a) ex vivo mit Zellen und/oder Zellgewebe.

2. Das Verfahren nach Anspruch 1, wobei die mindestens eine Nukleinsäure in der Zusammensetzung derart komplexiert ist, dass die negativen Ladungen der Nukleinsäure durch organische Kationen im Wesentlichen vollständig kompensiert sind.

3. Das Verfahren nach einem der vorhergehenden Ansprüche, wobei das Bereitstellen der Zusammensetzung in Schritt a) folgende Schritte umfasst:
a1) Lösen von Komplexen aus mindestens einer Nukleinsäure und mindestens einem organischen Kation in einem organischen Lösungsmittel oder einem Gemisch von organischen Lösungsmitteln, und gegebenenfalls
a2) Vermischen der in dem organischen Lösungsmittel oder Gemisch von organischen Lösungsmitteln gelösten Komplexe mit einer wässrigen Flüssigkeit.

4. Das Verfahren nach einem der vorhergehenden Ansprüche, wobei die Zusammensetzung nach a) eine Flüssigkeit ist, und wobei:
i) die Zellen und/oder Zellgewebe in einer Flüssigkeit vorliegen und das In-Kontakt-Bringen in Schritt b) das Mischen der beiden Flüssigkeiten umfasst,
ii) das In-Kontakt-Bringen in Schritt b) das Aufbringen der flüssigen Zusammensetzung auf Zellen und/oder Zellgewebe umfasst,
iii) das In-Kontakt-Bringen in Schritt b) das Resuspendieren von Zellen in der flüssigen Zusammensetzung umfasst, oder
iv) das In-Kontakt-Bringen in Schritt b) das Baden des Zellgewebes in der flüssigen Zusammensetzung umfasst.

5. Das Verfahren nach einem der vorhergehenden Ansprüche, wobei beim In-Kontakt-Bringen der Zusammensetzung nach a) ex vivo mit Zellen und/oder Zellgewebe die Konzentration des mindestens einen organischen Lösungsmittels, in dem die Komplexe aus mindestens einer Nukleinsäure und mindestens einem organischen Kation gelöst vorliegen, weniger als 10% (v/v), bevorzugt weniger als 1%, besonders bevorzugt weniger als 0,2% beträgt.

6. Das Verfahren nach einem der vorhergehenden Ansprüche, wobei das mindestens eine organische Lösungsmittel ein mit Wasser homogen mischbares organisches Lösungsmittel ist.

7. Das Verfahren nach einem der vorhergehenden Ansprüche, wobei das mindestens eine organische Lösungsmittel ausgewählt ist aus der Gruppe bestehend aus:
a) ein Alkohol,
b) DMSO, und
c) Glycolether wie z.B. Ethylenglykolmonobutylether.

8. Das Verfahren nach einem der vorhergehenden Ansprüche, wobei die Komplexe aus mindestens einer Nukleinsäure und mindestens einem organischen Kation in der Zusammensetzung gelöst vorliegen in einem Gemisch von:
a) zwei oder mehr Alkoholen,
b) einem oder mehr Alkoholen mit DMSO,
c) einem oder mehr Alkoholen mit einem oder mehr Glycolethern,
d) DMSO und einem oder mehr Glycolethern,
e) DMSO, einem oder mehr Alkoholen, und einem oder mehr Glycolethern,
f) einem Alkohol und einem Öl,
g) DMSO und einem Öl,
h) einem Glycolether und einem Öl,
i) Gemischen von, vorzugsweise homogenen, Lösungsmittelgemischen gemäß a) bis e) mit einem oder mehr Ölen, oder
j) Gemischen von, vorzugsweise homogenen, Lösungsmittelgemischen gemäß a) bis i) mit einer wässrigen Flüssigkeit.

9. Das Verfahren nach einem der vorhergehenden Ansprüche, wobei es sich bei der Nukleinsäure um DNA, RNA, oder DNA/RNA Hybride handelt.

10. Das Verfahren nach einem der vorhergehenden Ansprüche, wobei das In-Kontakt-Bringen in Gegenwart von Zellkulturmedium, Serum, Proteinen und/oder Antibiotika stattfindet.

11. Das Verfahren nach einem der vorhergehenden Ansprüche, wobei das mindestens eine organischen Kation eine Ammoniumverbindung ist.

12. Das Verfahren nach Anspruch 11, wobei das organische Kation eine quartäre Ammoniumverbindung ist.

13. Das Verfahren nach Anspruch 11 oder 12, wobei das organische Kation Cetyltrimethylammonium oder Benzalkonium ist.

14. Das Verfahren nach einem der vorhergehenden Ansprüche, wobei die Komplexe in der Zusammensetzung in einer homogenen Phase gelöst vorliegen.

15. Das Verfahren nach einem der vorhergehenden Ansprüche, wobei die Komplexe nicht innerhalb von Liposomen oder an die Oberfläche von Liposomen gebunden vorliegen.
